# EUROPÄISCHE PATENTSCHRIFT

(11) **EP 3 964 195 B1**
(45) Veröffentlichungstag und Bekanntmachung des Hinweises auf die Patenterteilung: **05.11.2025**
(21) Anmeldenummer: 20194557.3
(22) Anmeldetag: 07.09.2020
(51) Int. Cl.: A61K 8/99, A61Q 19/00, A61K 8/92, A61K 8/25, A23D 9/007, A23L 29/00, A23L 33/105, A23L 33/115, A23L 33/135

(54) **ZUSAMMENSETZUNG ZUR VERWENDUNG IN NAHRUNGSERGÄNZUNGSMITTELN UND/ODER KOSMETISCHEN PRODUKTEN UMFASSEND BACILLUS SUBTILIS**
COMPOSITION FOR USE IN DIETARY SUPPLEMENTS AND / OR COSMETIC PRODUCTS COMPRISING BACILLUS SUBTILIS
COMPOSITION DESTINÉE À L'UTILISATION DANS LES COMPLÉMENTS ALIMENTAIRES ET/OU LES PRODUITS COSMÉTIQUES COMPRENANT DU BACILLUS SUBTILIS

(43) Veröffentlichungstag der Anmeldung: 09.03.2022
(73) Patentinhaber: Institut Dr. Rilling Healthcare GmbH, 72124 Pliezhausen (DE)
(72) Erfinder: SEIPEL, Jochen, 72124 Pliezhausen (DE)
(74) Vertreter: Aera A/S

(56) Entgegenhaltungen:
- WO-A1-2020/150329
- DE-A1- 102016 114 687
- JP-A- 2016 041 645
- US-A1- 2020 108 106

## Beschreibung

Die vorliegende Erfindung betrifft eine Zusammensetzung umfassend Bacillus Subtilis, insbesondere zur Verwendung in Nahrungsergänzungsmitteln und/oder kosmetischen Produkten, ein Verfahren zur Herstellung der erfindungsgemäßen Zusammensetzung sowie ein Nahrungsergänzungsmittel als auch ein kosmetisches Produkt umfassend die erfindungsgemäße Zusammensetzung.

Nahrungsergänzungsmittel erfreuen sich immer öfter höherer Beliebtheit, um beispielsweise Mangelerscheinungen vorzubeugen. Insbesondere solche auf Basis von pflanzlichen Ölen und/oder Fetten in Kombination mit Silizium zeigen im Bereich des Knochenstoffwechsels, bei der natürlichen Kollagen- und Elastinsynthese, bei der Kräftigung der Struktur von Haaren und/oder Nägeln eine unterstützende Wirkung auf.

Silizium ist das zweithäufigste Element der Erde und Bestandteil zahlreicher Mineralien. Silizium kommt nie in freier Form vor, sondern überwiegend als Siliziumdioxid - auch bekannt unter dem Begriff Kieselsäure. Der menschliche Körper enthält etwa 20 mg Siliziumdioxid pro Kilogramm Körpergewicht. Da die Menge des Siliziumdioxids mit zunehmendem Alter abnimmt und der Körper es nicht selbst produzieren kann, muss dieses zwangsläufig mit der Nahrung und/oder entsprechende Nahrungsergänzungsmittel aufgenommen werden.

Hautpflegemittel und Nahrungsergänzungsmittel gekennzeichnet durch Gehalte an aus Stämmen von Bacillus Subtilis abgeleiteten Wirkstoffen sind aus DE 10 2016 114687 A1, WO 2020/150329 A1 und US 2020/108106 A1 bekannt. Ebenso werden vorteilhafte Wirkungen der Kieselsäure in der Hautpflege in JP 2016 041645 A beschrieben.

Ziel der vorliegenden Erfindung ist es daher eine bessere Zusammensetzung für Nahrungsergänzungsmittel und/oder für kosmetische Produkte, sowie ein verbessertes Nahrungsergänzungsmittel und/oder ein verbessertes kosmetisches Produkt bereitzustellen.

### Beschreibung der Erfindung

Erfindungsgemäß wird die Aufgabe durch eine Zusammensetzung mit den Merkmalen des Patentanspruchs 1, ein Verfahren mit den Merkmalen des Patentanspruchs 7, ein Nahrungsergänzungsmittel mit den Merkmalen des Patentanspruchs 9 sowie ein kosmetisches Produkt mit den Merkmalen des Patentanspruchs 11, gelöst.

Bezüglich der Zusammensetzung wird diese Aufgabe durch den Gegenstand des Patentanspruchs 1 gelöst. Diese Zusammensetzung umfasst mindestens 70 Gew.-% einer pflanzlichen Öl- und/oder Fettsubstanz, mindestens 0.5 Gew.-% Kieselsäure, und mindestens 0.05 Gew.-% Bacillus Subtilis ausgewählt aus einem der Stämme umfassend LA13030, CU1, HU58, DE 111, DSM 15544, PXN 21, Rosell-179 und/oder DSM 33619.

Überraschenderweise hat sich gezeigt, dass die Vermehrungsrate des Bakteriums Bacillus Subtilis ausgewählt aus einem der genannten Stämme in Gegenwart von Kieselsäure innerhalb von 24 Stunden signifikant gesteigert werden konnte, was sich besonders vorteilhaft auf das menschliche Immunsystem auswirkt.

In gleicher Weise sieht die Erfindung ein Verfahren zur Herstellung der erfindungsgemäßen Zusammensetzung vor, umfassend die Schritte:
- Bereitstellen von mindestens 70 Gew.-% einer pflanzlichen Öl- und/oder Fettsubstanz, mindestens 0.5 Gew.-% Kieselsäure, und mindestens 0.05 Gew.-% Bacillus Subtilis ausgewählt aus einem der Stämme umfassend LA13030, CU1, HU58, DE 111, DSM 15544, PXN 21, Rosell-179 und/oder DSM 33619; und
- Vermischen der Komponenten, so dass die Zusammensetzung erhalten wird.

In einem weiteren Aspekt betrifft die vorliegende Erfindung zudem ein Nahrungsergänzungsmittel sowie ein kosmetisches Produkt, umfassend die erfindungsgemäße Zusammensetzung.

Weitere vorteilhafte Ausgestaltungen der Erfindung sind in den abhängig formulierten Ansprüchen angegeben. Die in den abhängig formulierten Ansprüchen einzeln aufgeführten Merkmale sind in technologisch sinnvoller Weise miteinander kombinierbar und können weitere Ausgestaltungen der Erfindung definieren. Darüber hinaus werden die in den Ansprüchen angegebenen Merkmale in der Beschreibung näher präzisiert und erläutert, wobei weitere bevorzugte Ausgestaltungen der Erfindung dargestellt werden.

Der Bacillus Subtilis ist ausgewählt aus einem der Stämme umfassend LA13030, der ein deutscher Bacillus Subtilis Stamm ist; CU1, der ein französischer Bacillus Subtilis Stamm ist; HU58, der ein indischer/englischer Bacillus Subtilis Stamm ist; DE 111, der ein US-amerikanischer Bacillus Subtilis Stamm ist; DSM 15544, der ein japanischer Bacillus Subtilis Stamm ist; PXN 21, der ein englischer Bacillus Subtilis Stamm ist; Rosell-179, der ein kanadischer Bacillus Subtilis Stamm ist; und/oder DSM 33619. In einer besonders bevorzugten Ausführungsvariante umfasst die Zusammensetzung den Bacillus Subtilis Stamm DSM 33619, der auch unter der Bezeichnung DSM 21097 bekannt ist. Bei der Verwendung dieses Stammes hat sich überraschenderweise gezeigt, dass eine solche Zusammensetzung, also eine die mindestens 70 Gew.-% einer pflanzlichen Öl- und/oder Fettsubstanz, mindestens 0.5 Gew.-% Kieselsäure und mindestens 0.05 Gew.-% Bacillus Subtilis DSM 33619 umfasst, im Vergleich zu anderen Stämmen des Bacillus Subtilis, insbesondere dem Stamm ATCC 6633, eine antimikrobielle Wirkung gegenüber diversen Keimen aufweist.

In einer bevorzugten Ausführungsvariante kann die Zusammensetzung den Bacillus Subtilis in einer Menge von mindestens 0.06 Gew.-%, mehr bevorzugt von mindestens 0.07 Gew.-%, noch mehr bevorzugt von mindestens 0.08 Gew.-%, weiterhin bevorzugt von mindestens 0.09 Gew.-%, und am meisten bevorzugt von mindestens 0.1 Gew.-%. Hinsichtlich des maximalen Gehalts an dem Bacillus Subtilis ist die Zusammensetzung jedoch bevorzugt limitiert. Daher enthält die Zusammensetzung bevorzugt höchstens 5.0 Gew.-%, bevorzugt höchstens 2.0 Gew.-%, mehr bevorzugt höchstens 1.0 Gew.-%, und am meisten bevorzugt höchstens 0.5 Gew.-% Bacillus Subtilis.

Unter dem Begriff "Kieselsäure" werden im Sinne der vorliegenden Erfindung grundsätzlich die oligomeren und polymeren Kondensationsprodukte der Monokieselsäure Si(OH)₄ verstanden. Kieselsäure kann sowohl als uneinheitlich gebaute amorphe Kieselsäure als auch als kristallisierte Kieselsäure vorliegen. Im Sinne der vorliegenden Erfindung werden auch sog. Fällungskieselsäuren von dem Begriff der "Kieselsäure" verstanden. Unter Fällungskieselsäuren versteht man wässrige Lösungen der amorphen Kieselsäure, sog. Kieselsole, und angesäuerte, halbfeste Kieselsole, sog. Kieselgele. Darüber hinaus werden im Sinne der vorliegenden Erfindung auch die getrockneten Kieselgele unter dem Begrifft der "Kieselsäure" verstanden. Des Weiteren sind im Sinne der Erfindung zudem pyrogene Kieselsäuren von dem Begriff der "Kieselsäure" mitumfasst. Eine pyrogene Kieselsäure besteht im Wesentlichen aus einem amorphen SiO₂-Pulver, welches durch Flammenhydrolyse von Silanen oder SiCl₄ gewonnen wird.

In einer bevorzugt Ausführungsvariante ist die Kieselsäure daher eine gefällte und/oder eine pyrogene Kieselsäure.

In einer besonders vorteilhaften Ausführungsvariante umfasst die Zusammensetzung die Kieselsäure, insbesondere die gefällte, die pyrogene und/oder eine Mischung hiervon in einer Menge von mindestens 0.8 Gew.-%, mehr bevorzugt in einer Menge von mindestens 1.0 Gew.-%, noch mehr bevorzugt in einer Menge von mindestens 2.5 Gew.-%, weiterhin bevorzugt in einer Menge von mindestens 2.8 Gew.-%, und am meisten bevorzugt in einer Menge von mindestens 7.0 Gew.-%. Allerdings darf der Anteil nicht zu hoch sein. Daher beträgt der Gehalt an Kieselsäure bevorzugt höchstens 20 Gew.-%, mehr bevorzugt höchstens 15 Gew.-%, weiter bevorzugt höchstens 10 Gew.-%.

Die Kieselsäure weist vorteilhafterweise einen limitierten Wassergehalt nach DIN EN ISO 787-2 (1995-04) von höchstens 3.0 Gew.-% Wasser auf. Bevorzugt ist jedoch vorgesehen, dass die Kieselsäure einen Wassergehalt von 0.1 Gew.-% bis 0,5 Gew.-%, mehr bevorzugt einen Wassergehalt von 0.1 Gew.-% bis 0.3 Gew.-%, jeweils bezogen auf die Gesamtmenge Kieselsäure in der Zusammensetzung aufweist. Weiterhin bevorzugt weist die Kieselsäure eine BET-Oberfläche nach DIN ISO 9277 (2014-01) von 150 m²/g bis 250 m²/g, insbesondere von 160 m²/g bis 200 m²/g, auf.

In einer bevorzugten Ausführungsvariante des erfindungsgemäßen Verfahrens wird die Kieselsäure daher vor dem Vermischen getrocknet. Hierzu wird die Kieselsäure für mindestens 10 min einer Temperatur von maximal 200 °C ausgesetzt.

In einer vorteilhaften Ausführungsvariante umfasst die Zusammensetzung mindestens 80 Gew.-%, bevorzugt mindestens 90 Gew.-%, mehr bevorzugt mindestens 92 Gew.-%, noch mehr bevorzugt mindestens 97 Gew.-%, und am meisten bevorzugt mindestens 99 Gew.-% der pflanzlichen Öl- und/oder Fettsubstanz. In diesem Zusammenhang ist besonders bevorzugt vorgesehen, dass die pflanzliche Öl- und/oder Fettsubstanz ausgewählt ist aus der Reihe umfassend Olivenöl, Kokosöl, Mandelöl, Granatapfelkernöl, Mangobutter, Shea-Butter und/oder Mischungen hiervon.

Ganz besonders bevorzugt ist die pflanzliche Öl- und/oder Fettsubstanz ausgewählt aus der Reihe der mittelkettigen Triglyceride (MKT). Als mittelkettige Triglyceride werden im Sinne der vorliegenden Erfindung Mischungen verstanden, die üblicherweise als Hauptbestandteile 50 - 65 Gew.-% Caprylsäure (C8) und 30 - 45 Gew.-% Caprinäure (C10) enthalten. Weitere Bestandteile wie die Capronsäure (C6), Laurinsäure (C12) und Myristinsäure (C14) können in geringen Anteilen zugegegen sein. Typische aus dem Stand der Technik bekannte und kommerziell erhältliche mittelkettige Triglyceride sind beispielsweise unter den Handelsnamen Mygliol 812^{®} der Firma Sasol, Myritol 312^{®} der Firma Cognis und Tegosoft^{®} CT der Firma Evonik bekannt.

Die Verwendung dieser Öle und/oder Fette in kosmetischen Produkten als auch in Nahrungsergänzungsmitteln ist dem Fachmann grundsätzlich bekannt. Die mittelkettigen Triglyceride sind hilfreich bei der Gewichtsreduktion, verbessern die geistige- und körperliche Leistungsfähigkeit und schützen zudem vor verschiedenen Erkrankungen. Aufgrund ihrer Struktur weisen die mittelkettigen Triglyceride vorteilhafte physiologische Eigenschaften gegenüber herkömmlichen langkettigen Fettsäuren auf, und können daher einfacher vom Körper aufgenommen werden. So müssen diese MKTs nicht im Darm von Gallensäuren emulgiert werden, damit sie besser verstoffwechselt werden können. Sie bedürfen auch keiner Spaltung durch die Pankreaslipase, einem Enzym der Bauchspeicheldrüse. Die unterschiedlichen Verbindungen der mittelkettigen Triglyceride werden ohne Umgehung über das Lymphsystem direkt im Blut zur Leber transportiert, wo sie im Vergleich zu herkömmlichen Fetten bevorzugt oxidiert und vermehrt Ketonkörper bilden können, welche insbesondere vom Herzen und vom Gehirn als Energiequellen genutzt werden. Aus diesen Gründen werden die mittelkettigen Triglyceride bevorzugt bei der klinischen Ernährung zur diätischen Behandlung von verschiedenen Erkrankungen verwendet, wie beispielsweise dem Malabsorptionssyndrom, bei Lymphangiektasien, Morbus Whipple, Chylothorax, exokriner Pankreasinsuffizienz oder im Rahmen einer ketogenen Diät. Die ketogene Diät ist eine kohlenhydratlimitierte, protein- und energiebilanzierte und deshalb fettreiche Form der diätetischen Ernährung, bei welcher der Körper seinen Energiebedarf nur noch aus Fett und daraus im Körper aufgebautem Glukoseersatz, den namensgebenden Ketonkörpern bezieht. Eine ketogene Diät wird als Therapieverfahren vor allem bei Kindern mit pharmakoresistenter Epilepsie, Glukosetransporterstörung und Pyrovatdehydrogensasemangel eingesetzt. Neuere Forschungsergebnisse lassen auch eine Therapie bei Alzheimererkrankung möglich erscheinen.

Das Nahrungsergänzungsmittel bzw. das kosmetische Produkt kann die erfindungsgemäße Zusammensetzung in einer Menge von 10 bis 100 Gew.-%, mehr bevorzugt 20 bis 100 Gew.-% umfassen.

Sofern das Nahrungsergänzungsmittel die erfindungsgemäße Zusammensetzung zu 100 Gew.-% umfasst, ist vorteilhafterweise vorgesehen, dass die Zusammensetzung 97.1 Gew.-% MKT-Öl, 2.8 Gew.-% Kieselsäure und 0.1 Gew.-% Bacillus Subtilis des Stammes MM 40 enthält.

Weiterhin kann das Nahrungsergänzungsmittel und/oder das kosmetische Produkt weitere Mittel im Sinne der Nahrungsmittelergänzungsverordnung, wie Vitamine und Mineralstoffe, einschließlich Spurenelementen, umfassen.

Vorteilhafterweise sind die Vitamine und Mineralien ausgewählt aus der Gruppe umfassend Vitamin C, Vitamin D3, Vitamin K, Calcium- und Magnesiumsalzen sowie Mischungen hiervon.

Ein besonders bevorzugtes Vitamin ist das Vitamin C, welches ausgewählt ist aus der Gruppe umfassend L-Ascorbinsäure, Natrium-L-Ascorbat, Calcium-L-Ascorbat, Calium-L-Ascorbat, L-Ascorbyl-6-Palmitat sowie Mischungen hiervon. Ein weiteres bevorzugtes Vitamin ist Vitamin D in Form von Cholecalciferol oder Ergocalciferol. Weitere bevorzugte Vitamine sind Vitamin K, bevorzugt als Phyllochinon, Vitamin B3 und Vitamin E.

Gemäß einer vorteilhaften Ausführungsvariante kann das Nahrungsergänzungsmittel die erfindungsgemäße Zusammensetzung in einer Menge von 99.8 Gew.-% umfassen, wobei zusätzlich 0.1 Gew.-% Vitamin D3 in Form von Cholicalciferol und 0.1 Gew.-% Vitamin K in Form von Menachinon-7 enthalten sein können. Die Zusammensetzung kann vorzugsweise 97.1 Gew.-% MKT-ÖI, 2.8 Gew.-% Kieselsäure und 0.1 Gew.-% Bacillus Subtilis des Stammes MM 40 enthalten.

Das kosmetische Produkt kann die erfindungsgemäße Zusammensetzung in einer Menge von 35.4 Gew.-% umfassen, wobei zusätzlich 63.8 Gew.-% Mandelöl, 0.5 Gew.-% Vitamin E und 0.3 Gew.-% ätherisches Korianderöl enthalten sein können. Die Zusammensetzung kann vorzugsweise 98.9 Gew.-% MKT-ÖI, 1.0 Gew.-% Kieselsäure und 0.1 Gew.-% Bacillus Subtilis des Stammes MM 40 enthalten.

In einer anderen vorteilhaften Ausführungsvariante kann das kosmetische Produkt die erfindungsgemäße Zusammensetzung in einer Menge von 70 Gew.-% umfassen, wobei zusätzlich 15 Gew.-% Olivenöl, 10 Gew.-% ätherisches Minzöl und 5 Gew.-% ätherisches Oreganoöl enthalten sein können. Die Zusammensetzung kann vorzugsweise 97.1 Gew.-% MKT-ÖI, 2.8 Gew.-% Kieselsäure und 0.1 Gew.-% Bacillus Subtilis des Stammes MM 40 enthalten.

Besonders bevorzugte Mineralien sind Calcium- und Magnesiumsalze ausgewählt aus der Gruppe umfassend Calciumcarbonat, Calciumchlorid, Calciumsalze der Zitronensäure, Calciumgluconat, Calciumglycerophosphat, Calciumlactat, Calciumsalze der Orthophosphorsäure, Calciumhydroxid, Calciumoxid, Magnesiumacetat, Magnesiumcarbonat, Magnesiumchlorid, Magnesiumsalze der Zitronensäure, Magnesiumgluconat, Magnesiumglycerophosphat, Magnesiumsalze der Orthophosphorsäure, Magnesiumlactat, Magnesiumhydroxid, Magnesiumoxid und Magnesiumsulfat, Eisenoxid, Kaliumoxid, Natriumselenit, Selenomethionin sowie Mischungen hiervon.

In einer weiteren vorteilhaften Ausführungsvariante kann das Nahrungsergänzungsmittel die erfindungsgemäße Zusammensetzung in einer Menge von 23 Gew.-% umfassen, wobei zusätzlich 51.8 Gew.-% Calcium, 24.6 Gew.-% Magnesium und 0.6 Gew.-% Hydroxypropylmethylcellulose enthalten sein können. Die Zusammensetzung kann vorzugsweise 92.9 Gew.-% MKT-ÖI, 7.0 Gew.-% Kieselsäure und 0.1 Gew.-% Bacillus Subtilis des Stammes MM 40 enthalten.

In einer weiteren vorteilhaften Ausführungsvariante kann das Nahrungsergänzungsmittel die erfindungsgemäße Zusammensetzung in einer Menge von 23 Gew.-% umfassen, wobei zusätzlich 51.7 Gew.-% Calcium, 24.5 Gew.-% Magnesium, 0.6 Gew.-% Hydroxypropylmethylcellulose, 0.1 Gew.-% Vitamin D3 in Form von Cholicalciferol, und 0.1 Gew.-% Vitamin K in Form von Menachinon-7 enthalten sein können. Die Zusammensetzung kann vorzugsweise 92.9 Gew.-% MKT-ÖI, 7.0 Gew.-% Kieselsäure und 0.1 Gew.-% Bacillus Subtilis des Stammes MM 40 enthalten.

Weiterhin kann das Nahrungsergänzungsmittel und/oder das kosmetische Produkt Lebensmittelzusatzstoffe, wie insbesondere Farbstoffe, Konservierungsstoffe und/oder Antioxidantien enthalten. Diese werden eingesetzt, um Struktur, Geruch, Farbe und chemische und mikrobiologische Haltbarkeit des jeweiligen Produkts zu verbessern.

Konservierungsstoffe können beispielsweise ausgewählt sein aus der Gruppe Sorbinsäure, Kaliumsorbat, Calciumsorbat, Benzoesäure, Natriumbenzoat, Kaliumbenzoat, Calciumbenzoat, 4-Hydroxybenzoesäure-ethylester, Natrium-4-Hydroxybenzoesäure- ethylester, 4-Hydroxybenzoesäure-methylester, Schwefeldioxid, Natriumsulfit, Natriumhydrogensulfit, Natriumsdisulfit, Kaliumdisulfit, Calciumdisulfit, Calciumhydrogensulfit, Kaliumhydrogensulfit, Äpfelsäure, Fumarsäure und Lysozym sowie Mischungen hiervon.

Antioxidantien können beispielsweise ausgewählt sein aus der Gruppe umfassend Ascorbinsäure, Natriumascorbat, Calciumascorbat, Ascorbylpalmitat, Ascorbylsteatrat, Alpha-Tocopherol, Gamma-Tocopherol, Delta-Tocopherol, Propylgallat, Octylgallat, Dodecylgallat, Isoascorbinsäaure, Natriumisoascorbat, tert-Butylhydrochinon, Citronensäure, Phosphate, Posphorsäure, Bernsteinsäure und Rosmarinextrakt sowie Mischungen hiervon.

Weiterhin kann das erfindungsgemäße Nahrungsergänzungsmittel und/oder kosmetische Produkt Aromen umfassen, um dem jeweiligen Produkt einen besonderen Geruch und/oder Geschmack zu verleihen. Aromen können beispielsweise ausgewählt sein aus der Gruppe umfassend natürliche Aromastoffen, Aromaextrakte, thermisch gewonnene Reaktionsaromen, Raucharomen, Aromavorstufen und sonstige Aromen oder Mischungen hiervon. Bevorzugt umfasst das Nahrungsergänzungsmittel und/oder das kosmetische Produkt Aromen mit einer fruchtigen Geschmacksrichtung, beispielsweise ausgewählt aus der Reihe umfassend die Richtungen aus Orangen, Blutorangen, Grapefruit, Mandarinen, Zitronen und Kumquats. Besonders bevorzugt sind die Richtungen Orangen und Zitrone, insbesondere als Zitronenöl oder Orangenöl. Weitere bevorzugte Richtungen sind Beeren, Heidelbeeren, Himbeeren, Erdbeeren, Apfel, Birne, Trauben, Melonen, Koriander, Sandelholz, Fenchel, Weihrauch und Ingwer. Des Weiteren können Vanille- und Schokoladengeschmack bevorzugte Aromen sein.

Vorteilhafterweise ist das erfindungsgemäße Nahrungsergänzungsmittel und/oder das kosmetische Produkt frei von Aluminium.

### Beispiele

Die Erfindung sowie das technische Umfeld werden nachfolgend anhand von Beispielen näher erläutert. Es ist darauf hinzuweisen, dass die Erfindung durch die gezeigten Beispiele nicht beschränkt werden soll.

### Beispiel 1:

Um die antimikrobielle Wirkung von Bacillus Subtilis des Stammes MM 40 (DSM 21097) zu testen, wurde MH-Agar (Müller-Hinten-Agar) verwendet und mit einem MRSA Keim aus der Datenbank des Universitätsklinikums Lübeck (UKSH Lübeck) beimpft. Mit Hilfe von sterilen Testplättchen wurde Bacillus Subtilis DSM 33619 (DSM 21097) aufgetragen.

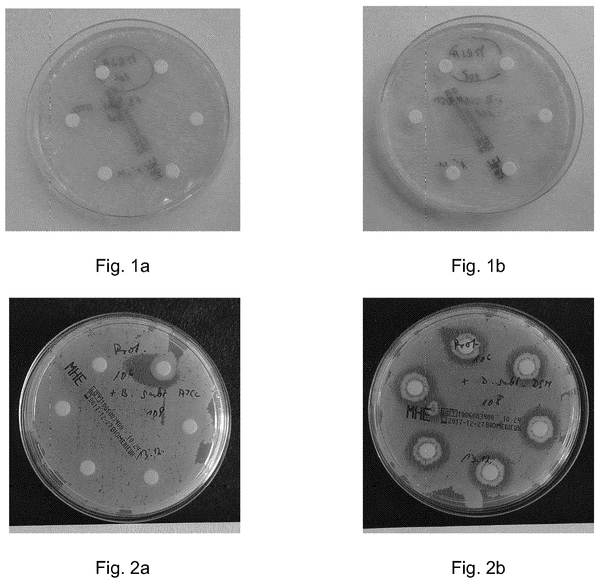

Wie anhand der Darstellung der Figur 1b erkennbar, konnte eine Hemmzone um den MRSA Keim identifiziert werden. Zur Kontrolle wurde der Stamm ATCC 6633 des Bacillus Subtillis verwendet, der keine Hemmzone aufwies (Figur 1a).

Weiterhin wurde mit dem gleichen Verfahren ein Vergleich auf den pathogenen Keim "Proteus Vulgaris" durchgeführt. Dabei konnte ebenfalls eine Hemmzone um den Keim Proteus Vulgaris festgestellt werden (Figur 2b). Zur Kontrolle wurde auch hier der Stamm ATCC 6633 des Bacillus Subtillis verwendet, der keine Hemmzone aufzeigte (Figur 2a).

### Beispiel 2:

Zur Untersuchung der Vermehrungsrate des Bakteriums Bacillus Subtilis des Stammes MM 40 (DSM 21097) wurde eine Zusammensetzung umfassend 97.1 Gew.-% MKT-ÖI, 2.8 Gew.-% Kieselsäure und 0.1 Gew.-% Bacillus Subtilis des Stammes MM 40 miteinander vermahlen und hiervon die Vermehrungsrate gemäß DIN EN ISO 4833-2:2014-05, § 64 LFGB L 00.00-88/2:2015-06 untersucht. Hierbei zeigte sich eine Steigerung der KBEs von 1*10⁵ KBE pro mL der Zusammensetzung auf 4*10¹⁰ KBE pro mL.

Eine Vergleichszusammensetzung ohne die Kieselsäure wies eine Vermehrungsrate von lediglich 1*10⁵ KBE pro mL der Zusammensetzung auf 1*10⁷ KBE pro mL der Zusammensetzung auf.

## Patentansprüche

1. Zusammensetzung umfassend:
- mindestens 70 Gew.-% einer pflanzlichen Öl- und/oder Fettsubstanz,
- mindestens 0.5 Gew.-% Kieselsäure, und
- mindestens 0.05 Gew.-% Bacillus Subtilis ausgewählt aus einem der Stämme umfassend LA13030, CU1, HU58, DE 111, DSM 15544, PXN 21, Rosell-179 und/oder DSM 33619.

2. Zusammensetzung nach Anspruch 1, umfassend mindestens 80 Gew.-%, bevorzugt mindestens 90 Gew.-%, mehr bevorzugt mindestens 92 Gew.-%, und noch mehr bevorzugt mindestens 97 Gew.-% der pflanzlichen Öl- und/oder Fettsubstanz.

3. Zusammensetzung nach Anspruch 1 oder 2, umfassend mindestens 0.8 Gew.-%, bevorzugt mindestens 1.0 Gew.-%, mehr bevorzugt mindestens 2.5 Gew.-%, und noch mehr bevorzugt mindestens 2.8 Gew.-% Kieselsäure.

4. Zusammensetzung nach einem der vorhergehenden Ansprüche, wobei die Kieselsäure eine gefällte und/oder eine pyrogene Kieselsäure ist.

5. Zusammensetzung nach einem der vorhergehenden Ansprüche, wobei die pflanzliche Öl- und/oder Fettsubstanz ausgewählt ist aus der Reihe umfassend Olivenöl, Kokosöl, Mandelöl, Granatapfelkernöl, Mangobutter, Shea-Butter und/oder Mischungen hiervon.

6. Zusammensetzung nach einem der vorhergehenden Ansprüche, wobei die pflanzliche Öl- und/oder Fettsubstanz ausgewählt ist aus der Reihe der mittelkettigen Triglyceride (MKT).

7. Verfahren zur Herstellung einer Zusammensetzung nach einem der vorhergehenden Ansprüche, umfassend die Schritte:
- Bereitstellen von mindestens 70 Gew.-% einer pflanzlichen Öl- und/oder Fettsubstanz, mindestens 0.5 Gew.-% Kieselsäure, und mindestens 0.05 Gew.-% Bacillus Subtilis ausgewählt aus einem der Stämme umfassend LA13030, CU1, HU58, DE 111, DSM 15544, PXN 21, Rosell-179 und/oder DSM 33619; und
- Vermischen der Komponenten, so dass die Zusammensetzung erhalten wird.

8. Verfahren nach Anspruch 7, wobei die Kieselsäure vor dem Vermischen getrocknet wird.

9. Nahrungsergänzungsmittel, umfassend eine Zusammensetzung nach einem der Ansprüche 1 bis 6.

10. Nahrungsergänzungsmittel nach Anspruch 9, wobei dieses frei von Aluminium ist.

11. Kosmetisches Produkt, umfassend eine Zusammensetzung nach einem der Ansprüche 1 bis 6.

## Claims

1. A composition, comprising:
- at least 70% by weight of a vegetable oil and/or fat substance,
- at least 0.5% by weight of silica, and
- at least 0.05% by weight of Bacillus subtilis selected from one of the strains comprising LA13030, CU1, HU58, DE 111, DSM 15544, PXN 21, Rosell-179 and/or DSM 33619.

2. The composition of claim 1, comprising at least 80% by weight, preferably at least 90% by weight, more preferably at least 92% by weight, and even more preferably at least 97% by weight of the vegetable oil and/or fat substance.

3. The composition of claim 1 or 2, comprising at least 0.8% by weight, preferably at least 1.0% by weight, more preferably at least 2.5% by weight, and even more preferably at least 2.8% by weight of silica.

4. The composition of any one of the preceding claims, wherein the silica is a precipitated and/or a pyrogenic silica.

5. The composition of any one of the preceding claims, wherein the vegetable oil and/or fat substance is selected from the group consisting of olive oil, coconut oil, almond oil, pomegranate seed oil, mango butter, shea butter, and/or mixtures thereof.

6. The composition of any one of the preceding claims, wherein the vegetable oil and/or fat substance is selected from the series of medium chain triglycerides (MCT).

7. A method for the preparation of a composition according to any one of the preceding claims, comprising the steps of:
- providing at least 70% by weight of a vegetable oil and/or fat substance, at least 0.5% by weight of silica, and at least 0.05% by weight of Bacillus subtilis selected from one of the strains comprising LA13030, CU1, HU58, DE 111, DSM 15544, PXN 21, Rosell-179 and/or DSM 33619; and
- mixing the components so that the composition is obtained.

8. The method of claim 7, wherein the silica is dried prior to mixing.

9. A food supplement comprising a composition of any one of claims 1 to 6.

10. A food supplement according to claim 9, wherein it is free of aluminum.

11. A cosmetic product comprising a composition of any one of claims 1 to 6.

## Revendications

1. Composition, comprenant :
- au moins 70 % en poids d'une substance huileuse et/ou grasse végétale,
- au moins 0,5 % en poids de silice, et
- au moins 0,05 % en poids de Bacillus Subtilis choisi parmi l'une des souches comprenant LA13030, CU1, HU58, DE 111, DSM 15544, PXN 21, Rosell-179 et/ou DSM 33619.

2. Composition selon la revendication 1, comprenant au moins 80 % en poids, de préférence au moins 90 % en poids, de manière davantage préférée au moins 92 % en poids, et de manière encore davantage préférée au moins 97 % en poids de la substance huileuse et/ou grasse végétale.

3. Composition selon la revendication 1 ou 2, comprenant au moins 0,8 % en poids, de préférence au moins 1,0 % en poids, de manière davantage préférée au moins 2,5 % en poids, et de manière encore davantage préférée au moins 2,8 % en poids d'acide silicique.

4. Composition selon l'une quelconque des revendications précédentes, dans laquelle l'acide silicique est un acide silicique précipité et/ou pyrogène.

5. Composition selon l'une quelconque des revendications précédentes, dans laquelle la substance huileuse et/ou grasse végétale est choisie dans le groupe comprenant l'huile d'olive, l'huile de coco, l'huile d'amande, l'huile de pépins de grenade, le beurre de mangue, le beurre de karité et/ou leurs mélanges.

6. Composition selon l'une quelconque des revendications précédentes, dans laquelle la substance huileuse et/ou grasse végétale est choisie dans la série des triglycérides à chaîne moyenne (MKT).

7. Procédé de préparation d'une composition selon l'une quelconque des revendications précédentes, comprenant les étapes consistant à :
- fournir au moins 70 % en poids d'une substance huileuse et/ou grasse végétale, au moins 0,5 % en poids d'acide silicique, et au moins 0,05 % en poids de Bacillus Subtilis choisi parmi l'une des souches comprenant LA13030, CU1, HU58, DE 111, DSM 15544, PXN 21, Rosell-179 et/ou DSM 33619 ; et
- mélanger les constituants pour obtenir la composition.

8. Procédé selon la revendication 7, dans lequel l'acide silicique est séché avant le mélange.

9. Complément alimentaire comprenant une composition selon l'une quelconque des revendications 1 à 6.

10. Complément alimentaire selon la revendication 9, qui est exempt d'aluminium.

11. Produit cosmétique comprenant une composition selon l'une quelconque des revendications 1 à 6.
